# EUROPEAN PATENT SPECIFICATION

(11) **EP 2 919 828 B1**
(45) Date of publication and mention of the grant of the patent: **18.10.2017**
(21) Application number: 13821138.8
(22) Date of filing: 15.11.2013
(51) Int. Cl.: A61M 1/16, A61M 1/36

(54) **BIOMEDICAL APPLIANCE**
BIOMEDIZINISCHE VORRICHTUNG
APPAREIL BIOMÉDICAL

(30) Priority: 16.11.2012 IT MO20120280
(43) Date of publication of application: 23.09.2015
(73) Proprietor: Eurosets S.r.l., 41036 Medolla (MO) (IT)
(72) Inventor: PETRALIA, Antonio, 41036 Medolla (IT); GHELLI, Nicola, 41036 Medolla (IT)
(74) Representative: Zoli, Filippo
(86) International application number: PCT/IB2013/002550
(87) International publication number: WO 2014/076549

(56) References cited:
- WO-A1-93/13852
- WO-A1-03/006139
- WO-A1-2004/089444
- US-A1- 2003 204 127
- US-A1- 2010 049 115

## Description

### Technical Field

The present invention relates to a biomedical appliance, in particular for blood oxygenation.

### Background Art

The use is known of oxygenators in the extracorporeal circulation of blood for bypassing the patient's heart and lungs during cardiac arrest and open-heart surgical operations, or to temporarily aid the patient's circulation and breathing in clinical conditions of acute heart failure and/or respiratory insufficiency (ECMO).

In particular, ECMO (Extracorporeal Membrane Oxygenator) is a non-therapeutic operation but which mechanically supports corporeal circulation and permits temporarily and partially replacing the heart and/or lung functions in order to permit their functional recovery. During this type of support, which can even continue for several days, it may be necessary to replace the oxygenating module because of its gradual loss of gaseous exchange capacity (oxygen and carbon dioxide).

In the event of having to replace the oxygenating module, to prevent air entering corporeal circulation, which would cause a serious condition of air embolism, it is absolutely necessary to perform a pre-filling of the new oxygenator, e.g., with a saline solution, so as to eliminate any air bubbles or pockets inside this.

The oxygenator pre-filling operation (commonly named priming) must be suitably performed by introducing a liquid substance from below to make sure the air bubbles come out. To date, this method has been generally performed by gravity or in a forced way.

In the first case, a liquid substance containment bag is positioned, to be introduced inside the oxygenator at a height greater than that of the oxygenator itself and this is connected to the latter by means of a supply line and a return line. The liquid substance contained in the bag then drops by gravity and gradually fills the oxygenator.

This solution is not however able to ensure the complete filling of the oxygenator and therefore the total elimination of any air bubbles in it due to the reduced pressure difference between the oxygenator inlet and outlet.

The forced filling of the oxygenator on the other hand envisages the use of an external pump, which is connected to the bag containing the liquid substance to be introduced into the oxygenator to increase the supply pressure and thus ensure the complete filling of the oxygenator itself and the total removal of the air from the various transit sections.

This filling method also has a number of drawbacks.

In fact, the use of an external pump involves high costs, tied to the purchase and use of the pump itself, and calls for adequate space for positioning, in a highly critical environment like reanimation.

Furthermore, because such health facility has a limited number of pumps usable for this purpose (commonly called heart-lung machines), it follows that the pump itself may also not be available at the time it is required, thus prejudicing the correct and prompt replacement of the oxygenator.

Biomedical appliances suitable for priming a device or similar are known by WO 93/13852 and by US 2010/0049115.

WO 93/13852 discloses an appliance suitable to fill a device for treating blood with a liquid. Such appliance comprises a bag containing a priming fluid and provided with a unique mouth by which the fluid is supplied to the device and return from the same into the bag.

US 2010/0049115 discloses an apparatus and a method for priming an arterial and a venous line.

### Description of the Invention

The main aim of the present invention is to provide a biomedical appliance which allows to overcome the drawbacks of prior art.

As part of this aim, one object of the present invention is to allow the complete and correct filling of an oxygenating module with a liquid substance, thereby ensuring the complete removal of any air bubbles inside it, and at the same time cutting the costs of investment and use.

One object of the present invention is to speed up the preparation times of the oxygenating module, while at the same time remaining at the side of the patient's bed.

Another object of the present invention is to provide an appliance which is small and easy to use.

Another object of the present invention is to provide a biomedical appliance which allows to overcome the mentioned drawbacks of the state of the art within the ambit of a simple, rational, easy and effective to use as well as low cost solution.

The objects mentioned above are achieved by the present biomedical appliance according to claim 1.

### Brief Description of the Drawings

Other characteristics and advantages of the present invention will become more evident from the description of a preferred, but not sole, embodiment of a biomedical appliance, illustrated purely as an example but not limited to the annexed drawings in which:
Figure 1 is a schematic view of an appliance according to the invention, in a first embodiment;
Figure 2 is a schematic view of an appliance according to the invention, in an alternative embodiment.

### Embodiments of the Invention

With particular reference to such figures, globally indicated by reference numeral 1 is an appliance comprising a device for the oxygenation 2 of blood and a circuit 20 for the pre-filling of the device 2.

The device 2 is of the type known to the technician in the sector and is meant to be fitted along an extracorporeal blood circulation line to oxygenate the blood arriving from the patient and reintroduce it back into the patient once it has been oxygenated.

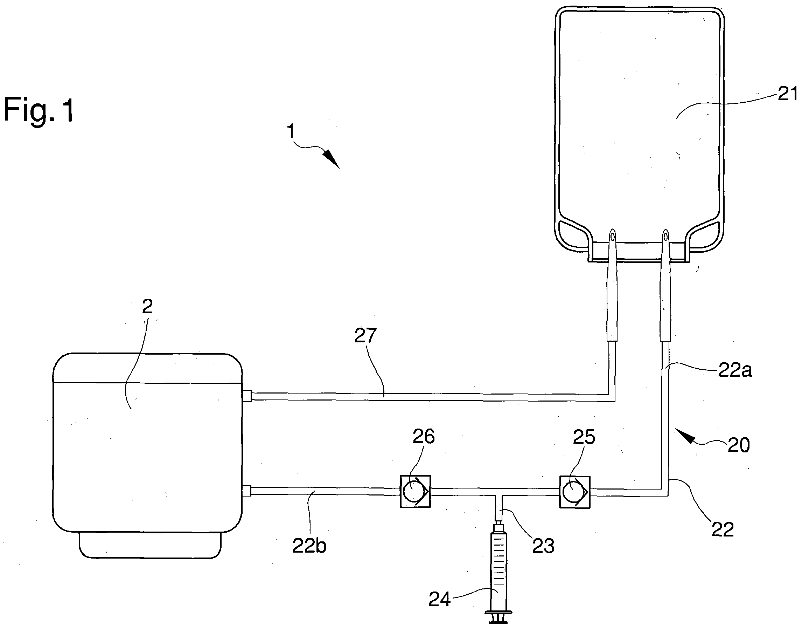

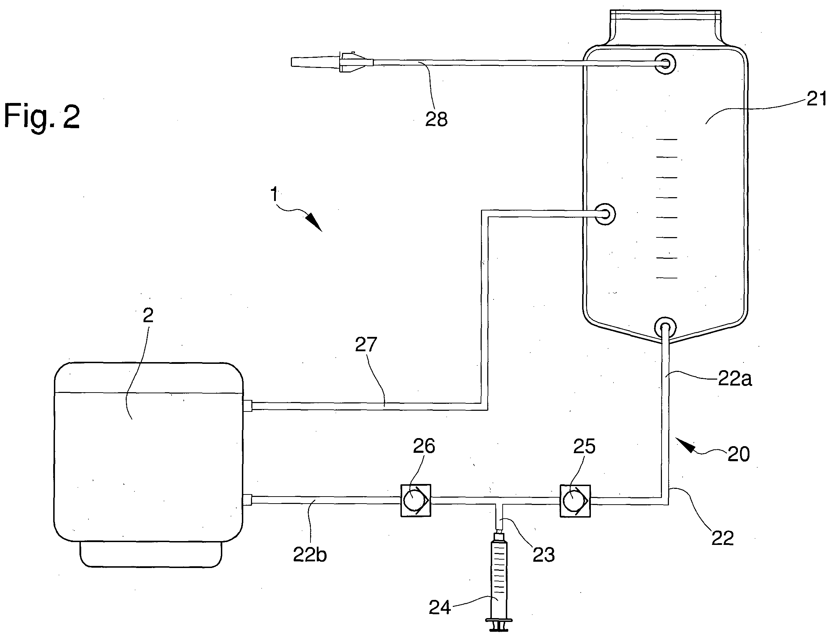

The device 2, shown schematically in the figures 1 and 2, comprises a containment body defining an oxygenation chamber meant to be crossed by the blood arriving from the patient and inside which is made to circulate a work gas suitable for yielding oxygen to the blood.

The containment body therefore has at least an inlet fitting, through which the blood arriving from the patient is meant to be introduced into the oxygenation chamber, and an outlet fitting, through which the oxygenated blood is meant to exit before being reintroduced into the patient him/herself.

It is further known that the device 2 also comprises an inlet channel and an outlet channel for a work gas, not shown in the illustrations inasmuch as known to the technician in the sector and not relevant for the purposes of the present invention, meant to yield oxygen to the blood inside the oxygenation chamber.

According to the invention, the circuit 20 comprises at least a bag 21 for containing a liquid substance to be introduced into the device 2 to eliminate the air contained inside it before the device itself is positioned along the extracorporeal blood circulation line connected to the patient. The liquid substance contained in the bag 21 is e.g. made up of a saline solution.

The bag 21 has at least a first and a second mouth, distinct from one another, to allow the liquid substance to come out and reinfuse respectively.

The circuit 20 then comprises at least a supply line 22 which has a first extremity communicating with the first mouth of the bag 21 and a second extremity connected to the inlet fitting of the device 2. The first extremity of the supply line 22 is preferably associated with the bag 21 in correspondence to the bottom of same.

The supply line 22 therefore has a first section 22a placed between its first extremity and the pumping means 24 and a second section 22b placed between the pumping means themselves and its second extremity.

In the preferred embodiments shown in the illustrations, from the supply line 22 branches out at least a shunt line 23 placed between the bag 21 and the device 2. The branch line 23 is therefore placed between the first and the second extremities of the supply line 22. In this embodiment, the first section 22a is placed between the first extremity of the supply line 22 and the shunt line 23 and the second section 22b is placed between the shunt line 23 and the second extremity of the supply line itself.

The appliance 1 then also comprises pumping means 24 of the manual type arranged along the supply line 22, upstream of the device 2 with respect to the direction of forward movement of the liquid substance inside the supply line itself and which can be operated to suction the liquid substance from the bag 21 and convey the liquid substance suctioned this way into the device 2. The pumping means 24 consist, e.g., of a syringe.

In the preferred embodiments shown in the illustrations, the pumping means 24 are associated with the branch line 23.

Along the supply line 22, valve means 25, 26 are arranged that can be operated during the suction and conveying of the liquid substance to prevent at least the liquid substance from flowing from the device 2 towards the pumping means 24 and from the pumping means themselves towards the bag 21 respectively.

Advantageously, the valve means 25, 26 comprise first valve means 25 and second valve means 26 positioned on opposite sides of the pumping means 24.

In the embodiments shown in the illustrations, the first valve means 25 are placed between the bag 21 and the shunt line 23 and the second valve means 26 are placed between the shunt line itself and the device 2.

The first and second valve means 25 and 26 are therefore arranged along the first and second sections 22a and 22b, respectively, of the supply line 22.

More in particular, the valve means 25,26 can be operated to obstruct the second section 22b, at least in the direction of the shunt line 23, during the suction of the liquid substance from the bag 21 by means of the pumping means 24 and to obstruct the first section 22a, at least in the direction of the bag 21, during the conveying of the liquid substance towards the device 2 by means of the pumping means themselves.

As shown in the illustrations 1 and 2, the first and second valve means 25 and 26 comprise a respective unidirectional valve.

More in detail, the first valve means 25 comprise a unidirectional valve suitable for allowing only the flow of the liquid substance along the first section 22a towards the pumping means 24. Such unidirectional valve 25 thus prevents the liquid substance from flowing in the opposite direction, i.e., from the shunt line 23 towards the bag 21 irrespective of the pressure in its ends.

Similarly, the second valve means 26 comprise a unidirectional valve suitable for only allowing the flow of the liquid substance along the second section 22b towards the device 2. Such unidirectional valve 26 thus prevents the liquid substance from flowing from the device 2 towards the shunt line 23 irrespective of the pressure in its ends.

The unidirectional valves 25 and 26 start automatically to allow the flow of the liquid substance by effect of a difference in pressure at their ends corresponding to the relative setting pressure.

For the technician in the sector, it is easy to appreciate how the first solution described, wherein the valve means 25, 26 are composed of unidirectional valves, is preferable because it provides better operating liability because the operation of the valves themselves is automatic and therefore does not depend on the operator.

The circuit 20 then comprises at least a return line 27, distinct from the supply line 22, communicating on one side with the second mouth of the bag 21 and on the opposite side with the outlet fitting of the device 2. The return line 27 is therefore suitable for reintroducing inside the bag 21 the liquid substance that comes out of the device 2 along with the air contained in same. The circuit 20 thus associated with the bag 21 and the device 2 therefore defines a closed path. Advantageously, as represented in the embodiment of figure 2, the return line 27 is associated with the bag 21 at a greater height than the supply line 22.

More in particular, the second mouth of the bag 21 is arranged at a greater height than the first mouth of the bag itself, which means that the extremity of the return line 27 associated with the bag 21 is arranged higher up, e.g., in correspondence to the intermediate area of the bag itself, compared to the first extremity of the supply line 22. This layout of the connection between the return line 27 and the bag 21 permits ensuring that any air bubbles which are reintroduced into the bag 21 through the return line 27 are collected up in the top part of the bag itself, thus avoiding these mixing with the liquid substance from time to time suctioned along the first section 22a.

In the embodiment shown in figure 2, the circuit 20 also comprises a filling line 28. Such filling line 28 is associated on one end with the bag 21, while on the opposite end it can be associated with a vessel for containing the liquid substance to be introduced into the device 2.

The filling line 28 therefore comprises a free extremity, having a relative connector, which is associable with the above-mentioned vessel in order to fill the bag 21.

The operation of the present invention is as follows.

First of all, the circuit 20 is connected to the device 2 by associating the supply line 22 and the return line 27 to the inlet fitting and to the outlet fitting respectively of the device itself.

Then the second section 22b is obstructed and the liquid substance suctioned from the bag 21 by means of the pumping means 24.

In the first embodiment shown in the figures 1 and 2, the obstruction of the second section 22b is done by the unidirectional valve 26, which prevents the flow of any liquid substance already contained in the device 2 towards the pumping means 24.

In the second embodiment described above, the operator must instead intervene manually on the taps arranged along the sections 22a and 22b respectively before the suction phase and before the liquid substance conveying stage. More in detail, in this second embodiment, the operator must close the tap arranged along the second section 22b before suctioning the liquid substance with the pumping means 24, so as to prevent any liquid substance contained or already introduced into the device 2 from being suctioned towards the pumping means themselves. Suitably, in this phase, the tap arranged along the first section 22a must be in open position.

Similarly, in the third embodiment, the operator must intervene manually only on the three-way tap bringing it to the first position before the suction phase of the liquid substance from the bag 21.

After suctioning the liquid substance from the bag 21, the second section 22b is then opened and the first section 22a obstructed in such a way as to allow the liquid substance contained in the pumping means 24 to flow towards the device 2 and at the same time prevent it from returning into the bag 21.

These phases are automatically performed by the unidirectional valves 25 and 26 in the first embodiment, while they must be carried out manually by the operator in the second and third embodiments described above.

More in particular, in the second embodiment, the operator must again intervene on the valve means, bringing the taps arranged along the sections 22a and 22b to closed and open position respectively, in such a way as to allow the liquid substance contained in the pumping means 24 to flow towards the device 2 and at the same time prevent it from returning into the bag 21.

In the third embodiment, on the other hand, the operator must intervene manually only on the three-way tap bringing it to the second position before the conveying phase of the liquid substance inside the device 2.

Subsequently, the suctioned liquid substance is conveyed inside the device 2 by again intervening on the pumping means 24 but in the opposite way with respect to the suction phase.

The phases of suctioning and conveying of the liquid substance by means of the pumping means must be repeated until the device 2 is completely filled, i.e., until the liquid substance introduced into it reaches the return line 27 which closes the circuit 20.

More in particular, the filling of the device 2 with the liquid substance introduced through the supply line 22 causes the air contained in the device itself to come out and this is therefore conveyed inside the bag 21 along with the liquid substance, through the return line 27.

Inside the bag 21 therefore, the liquid substance is separated from the air initially contained inside the device 2. Such separation is made easier in the case of the second mouth of the bag 21 being arranged at a greater height than that of the first mouth, inasmuch as the air is directly reintroduced into the top part of the bag itself.

For a technician in the sector, the operation of the present invention in the second and in the third embodiments described above is easy to appreciate.

It has in fact been ascertained how the described invention achieves the proposed objects and in particular the fact is underlined that it permits easily and reliably filling the blood oxygenation device before this is fitted in the extracorporeal blood circulation line and in particular ensures the complete removal of the air contained in the device itself.

The appliance according to the invention does in fact ensure the complete filling of the oxygenation device thanks to the use of manual pumping means which permit applying a considerably greater pressure compared to gravity filling. At the same time, the simple construction of the circuit according to the invention considerably cuts operating costs and overall dimensions compared to the use of an external pump and it is, furthermore, always available for use.

Moreover, once positioned along an extracorporeal circulation line, the oxygenation device filled by means of the circuit according to the invention is in fact already ready to use thereby reducing replacement times compared to known methods and therefore also the duration of the interruption of the perfusion to the patient.

## Claims

1. Biomedical appliance (1), comprising:
- a device (2) for the oxygenation of blood having at least an inlet fitting and at least an outlet fitting for blood;
- a circuit (20) for the pre-filling of said device (2) for the oxygenation;
- at least a bag (21) for containing a liquid substance having at least a first and a second mouths, distinct from one another, to allow the liquid substance to come out and reinfuse, respectively;
where said circuit (20) comprises:
- at least a supply line (22) communicating on one side with the first mouth of said bag (21) and on the other side with the inlet fitting of the device (2) for the oxygenation;
- manual pumping means (24) arranged along said supply line (22) and which can be operated to suction the liquid substance from said bag (21) and to convey the liquid substance suctioned this way into the device (2) for the oxygenation;
- at least a shunt line (23) which branches out from said supply line (22) and placed between the first mouth of said bag (21) and the inlet fitting of said device (2) for the oxygenation, and that said pumping means (24) are associated with said shunt line (23);
and wherein said circuit (20) comprises
- valve means (25, 26) arranged only along said supply line (22) and which can be operated during the suction and conveying of the liquid substance to prevent at least the substance itself from flowing from the device (2) for the oxygenation towards said pumping means (24) and from the latter towards said bag (21), respectively;
where said valve means (25, 26) comprise first and second valve means arranged on opposite sides of said pumping means (24), said first valve means (25) comprising an unidirectional valve placed between said bag (21) and said shunt line (23) to prevent the liquid substance from flowing towards the bag itself and said second valve means (26) comprising an unidirectional valve placed between said shunt line (23) and said device (2) for the oxygenation to prevent the liquid substance from flowing towards the shunt line itself;
- at least a return line (27), distinct from said supply line (22), communicating on one side with the outlet fitting of the device (2) for the oxygenation and on the other side directly connected with the second mouth of said bag (21), said return line (27) being suitable for reintroducing the liquid substance that comes out of said device (2) for the oxygenation, along with the air contained in same, inside said bag (21).

2. Appliance (1) according to claim 1, **characterized by** the fact that said pumping means (24) are of the type of a syringe.

3. Appliance (1) according to one or more of the preceding claims, **characterized by** the fact that said first mouth is defined in correspondence to the bottom of said bag (21).

4. Appliance (1) according to one or more of the preceding claims, **characterized by** the fact that said second mouth is defined at a greater height than said first mouth.

5. Appliance (1) according to claim 4, **characterized by** the fact that said second mouth is defined in correspondence to a median area of said bag (21).

6. Appliance (1) according to one or more of the preceding claims, **characterized by** the fact that it comprises at least a filling line (28) associated with said bag (21) and connectable to a vessel for collecting the liquid substance to be introduced into the bag itself.

7. Appliance (1) according to claim 6, **characterized by** the fact that said filling line (28) is associated with said bag (21) in correspondence to its upper portion.

## Patentansprüche

1. Biomedizinische Vorrichtung (1), umfassend
- eine Einrichtung (2) zur Oxygenierung von Blut mit mindestens einem Einlassfitting und mindestens einem Auslassfitting für Blut;
- einen Kreislauf (20) zum Vorfüllen der Einrichtung (2) für die Oxygenierung;
- mindestens einen Beutel (21) zum Enthalten einer flüssigen Substanz mit mindestens einer ersten und einer zweiten, voneinander getrennten Öffnung, die das Austreten bzw. das erneute Eintragen der flüssigen Substanz erlauben;
wobei der Kreislauf (20) umfasst:
- mindestens eine Versorgungsleitung (22), die auf einer Seite mit der ersten Öffnung des Beutels (21) und auf der anderen Seite mit dem Einlassfitting der Einrichtung (2) für die Oxygenierung in Verbindung steht;
- manuelle Pumpmittel (24), die entlang der Versorgungsleitung (22) angeordnet sind und die zum Ansaugen der flüssigen Substanz aus dem Beutel (21) und zum Überführen der derart angesaugten flüssigen Substanz in die Einrichtung (2) für die Oxygenierung bedienbar sind;
- mindestens eine Abzweigleitung (23), die von der Versorgungsleitung (22) abzweigt und zwischen der ersten Öffnung des Beutels (21) und dem Einlassfitting der Einrichtung (2) für die Oxygenierung angeordnet ist, wobei die Pumpmittel (24) mit der Abzweigleitung (23) verbunden sind;
und wobei der Kreislauf (20) umfasst:
- Ventilmittel (25, 26), die nur entlang der Versorgungsleitung (22) angeordnet sind und die während des Ansaugens und Überführens der flüssigen Substanz bedienbar sind, um zumindest zu verhindern, dass die Substanz von der Einrichtung (2) für die Oxygenierung zu den Pumpmitteln (24) fließt bzw. von letzterer zu dem Beutel (21);
wobei die Ventilmittel (25, 26) erste und zweite Ventilmittel umfassen, die an gegenüberliegenden Seiten der Pumpmittel (24) angeordnet sind, wobei das erste Ventilmittel (25) ein Rückschlagventil umfasst, das zwischen dem Beutel (21) und der Abzweigleitung (23) angeordnet ist, um das Fließen der flüssigen Substanz zum Beutel zu verhindern, und wobei das zweite Ventilmittel (26) ein Rückschlagventil umfasst, das zwischen der Abzweigleitung (23) und der Einrichtung (2) für die Oxygenierung angeordnet ist, um das Fließen der flüssigen Substanz zur Abzweigleitung zu verhindern;
- mindestens eine Rückstromleitung (27), die von der Versorgungsleitung (22) verschieden ist und die auf einer Seite mit dem Auslassfitting der Einrichtung (2) für die Oxygenierung in Verbindung steht und auf der anderen Seite direkt mit der zweiten Öffnung des Beutels (21) verbunden ist, wobei die Rückstromleitung (27) zum erneuten Eintragen der flüssigen Substanz, die aus der Einrichtung (2) für die Oxygenierung kommt, zusammen mit der darin enthaltenen Luft in den Beutel (21) geeignet ist.

2. Vorrichtung (1) noch Anspruch 1, **dadurch gekennzeichnet, dass** die Pumpmittel (24) vom Typ einer Spritze sind.

3. Vorrichtung (1) nach einem oder mehreren der vorhergehenden Ansprüche, **dadurch gekennzeichnet, dass** die erste Öffnung korrespondierend zum unteren Teil des Beutels (21) festgelegt ist.

4. Vorrichtung (1) nach einem oder mehreren der vorhergehenden Ansprüche, **dadurch gekennzeichnet, dass** die zweite Öffnung oberhalb der ersten Öffnung festgelegt ist.

5. Vorrichtung (1) nach Anspruch 4, **dadurch gekennzeichnet, dass** die zweite Öffnung korrespondierend zu einem mittleren Bereich des Beutels (21) festgelegt ist.

6. Vorrichtung (1) nach einem oder mehreren der vorhergehenden Ansprüche, **dadurch gekennzeichnet, dass** sie mindestens eine Füllleitung (28) umfasst, die mit dem Beutel (21) verbunden und mit einem Gefäß zum Sammeln der in den Beutel einzutragenden flüssigen Substanz verbindbar ist.

7. Vorrichtung (1) nach Anspruch 6, **dadurch gekennzeichnet, dass** die Füllleitung (28) mit dem Beutel (21) korrespondierend zu dessen oberem Abschnitt verbunden ist.

## Revendications

1. Appareil biomédical (1), comprenant :
- un dispositif (2) pour l'oxygénation du sang ayant au moins un raccord d'entrée et au moins un raccord de sortie pour le sang ;
- un circuit (20) pour le pré-remplissage dudit dispositif (2) pour l'oxygénation ;
- au moins un sac (21) pour contenir une substance liquide ayant au moins des première et seconde embouchures, distinctes l'une de l'autre, pour permettre à la substance liquide de sortir et d'être reperfusée, respectivement ;
où ledit circuit (20) comprend :
- au moins une conduite d'alimentation (22) communiquant, sur un côté, avec la première embouchure dudit sac (21) et, sur l'autre côté, avec le raccord d'entrée du dispositif (2) pour l'oxygénation ;
- un moyen de pompage manuel (24) agencé le long de ladite conduite d'alimentation (22) et qui peut être actionné pour aspirer la substance liquide depuis ledit sac (21) et pour acheminer la substance liquide aspirée de cette manière dans le dispositif (2) pour l'oxygénation ;
- au moins une conduite de dérivation (23) qui bifurque depuis ladite conduite d'alimentation (22) et placée entre la première embouchure dudit sac (21) et le raccord d'entrée dudit dispositif (2) pour l'oxygénation, et que ledit moyen de pompage (24) est associé à ladite conduite de dérivation (23) ;
et dans lequel ledit circuit (20) comprend
- des moyens de valve (25, 26) agencés uniquement le long de ladite conduite d'alimentation (22) et qui peuvent être actionnés durant l'aspiration et l'acheminement de la substance liquide pour empêcher au moins la substance elle-même de s'écouler depuis le dispositif (2) pour l'oxygénation vers ledit moyen de pompage (24) et depuis ce dernier vers ledit sac (21), respectivement ;
où lesdits moyens de valve (25, 26) comprennent des premier et second moyens de valve agencés sur des côtés opposés dudit moyen de pompage (24), ledit premier moyen de valve (25) comprenant une valve unidirectionnelle placée entre ledit sac (21) et ladite conduite de dérivation (23) pour empêcher la substance liquide de s'écouler vers le sac lui-même et ledit second moyen de valve (26) comprenant une valve unidirectionnelle placée entre ladite conduite de dérivation (23) et ledit dispositif (2) pour l'oxygénation pour empêcher la substance liquide de s'écouler vers la conduite de dérivation elle-même ;
- au moins une conduite de retour (27), distincte de ladite conduite d'alimentation (22), communiquant, sur un côté, avec le raccord de sortie du dispositif (2) pour l'oxygénation et, sur l'autre côté, directement connectée à la seconde embouchure dudit sac (21), ladite conduite de retour (27) étant appropriée pour réintroduire la substance liquide qui sort dudit dispositif (2) pour l'oxygénation, avec l'air contenu dans celui-ci, à l'intérieur dudit sac (21).

2. Appareil (1) selon la revendication 1, **caractérisé par le fait que** ledit moyen de pompage (24) est du type d'une seringue.

3. Appareil (1) selon une ou plusieurs des revendications précédentes, **caractérisé par le fait que** ladite première embouchure est définie en correspondance avec le fond dudit sac (21).

4. Appareil (1) selon une ou plusieurs des revendications précédentes, **caractérisé par le fait que** ladite seconde embouchure est définie à une hauteur plus élevée que celle de ladite première embouchure.

5. Appareil (1) selon la revendication 4, **caractérisé par le fait que** ladite seconde embouchure est définie en correspondance avec une zone médiane dudit sac (21).

6. Appareil (1) selon une ou plusieurs des revendications précédentes, **caractérisé par le fait qu'**il comprend au moins une conduite de remplissage (28) associée audit sac (21) et pouvant être connectée à un récipient pour collecter la substance liquide à introduire dans le sac lui-même.

7. Appareil (1) selon la revendication 6, **caractérisé par le fait que** ladite conduite de remplissage (28) est associée audit sac (21) en correspondance avec sa partie supérieure.
